# EUROPEAN PATENT APPLICATION

(11) **EP 4 049 536 A1**
(43) Date of publication of application: **31.08.2022**
(21) Application number: 22159043.3
(22) Date of filing: 26.02.2022
(51) Int. Cl.: A23B 7/02, A23B 7/024, A23B 7/04, A23B 7/055, A61K 8/9789, A61K 9/19, A61K 36/185, A23N 15/00, A23L 33/105

(54) **METHOD FOR THE TREATMENT OF CANNABIS SATIVA L**

(30) Priority: 26.02.2021 IT 202100004595
(71) Applicant: CanapaLife S.r.l., 35137 Padova (IT)
(72) Inventor: Garro, Alvaro, 35142 Padova (IT); Puggioni, Paolo, 35010 Campodoro (IT)
(74) Representative: Pelanda, Paolo

(57) **Abstract**

A method for processing Cannabis Sativa L., comprising a step a) of destemming of the flowers of Cannabis Sativa L., a step b) of freezing of the flowers Cannabis Sativa L., a step c) of trimming of the flowers of Cannabis Sativa L., a step d) of drying of the flowers of Cannabis Sativa L.. The destemming step a) is suited to promote the separation of the flowers of Cannabis Sativa L. from the branches. The trimming step c) is suited to promote the removal of the outermost leaves of each flower of Cannabis Sativa L. and the step d) of drying of the flowers of Cannabis Sativa L. is carried out in high vacuum environmental conditions in such a way as to promote the sublimation into vapour of the water contained in the flowers of Cannabis Sativa L..

## Description

### Field of application of the invention

The present invention relates to the technical field of intensive cultivation of medicinal plants and its subject is a method for the processing of Cannabis Sativa L.

### State of the art

As it is well known, the technical field of cultivation of medicinal plants has recently expanded with the Cannabis Sativa L. species, from whose flower it is possible to extract substances used for therapeutic and cosmetic purposes.

In particular, Cannabis Sativa L. is rich in *cannabidiol* or *CBD,* a molecule with different therapeutic properties and mainly used in substances for relaxation purposes.

In addition, CBD appears to have beneficial effects in the care and treatment of anxiety (antipsychotic), nausea and vomiting (antiemetic) and seizures (anticonvulsant).

Alternatively, the CBD molecule, if suitably processed, can be used in energizing beverages.

Unlike the cannabinoid THC, CBD has no psychoactive properties.

In addition, some varieties of Cannabis Sativa L. are naturally free of THC and this makes the cultivation, processing and administration of the extracts of this plant completely legal.

From a commercial standpoint, the demand for extracts from Cannabis Sativa L. has recently experienced a sudden growth; in particular, the consumption of products containing substances generated or extracted from this plant has spread very quickly among the population, especially among young people.

The cannabidiol CBD is mainly present in the Cannabis Sativa L. flower; for this reason, the transformation process of this plant is aimed at obtaining the maximum production of these flowers.

However, it is well known that the harvesting cycle of Cannabis Sativa L. flowers is rather long and complex, and for this reason some processing techniques have been developed over time which are aimed at maximizing the production and the yield of the extracts contained in these flowers.

The traditional processing method of Cannabis Sativa L. involves the steps of harvesting of the branches containing the flowers, destemming, trimming, drying, and storage.

The destemming step ("de" + "stem") involves the separation of the flowers from the branches while the trimming step involves the removal of the apical leaves of the flower located at its outermost part (thus obtaining the "heart" of the flower itself).

The drying step is generally carried out in special environments such as rooms, greenhouses or drying ovens in non-ventilated and/or ventilated conditions, generally with temperatures between 15°C and 40°C.

In such environments, dehydrating or dehumidifying devices can be used to lower the humidity level so as to promote the extraction of water from the flowers and promote their drying.

The drying step is traditionally performed under atmospheric pressure conditions.

The trimming step is generally carried out when the product is still "green" (with the fresh product) or when it is "dry" (with the already dried product).

While the most common methods for processing Cannabis Sativa L. guarantee good yields from a quantitative point of view, they are not always able to ensure high quality in all the transformation processes the flowers are subjected to, in particular due to the onset of mold during the drying process and/or loss of the original colour.

For this reason, the traditional methods of harvesting and processing the flowers of Cannabis Sativa L. do not allow the quality of the product to be controlled during the execution of the different steps and, even worse, these methods do not allow to obtain - at the end of the processing steps - a finished product (consisting of flowers) with uniform and homogeneous characteristics intended to maximize the amount of CBD contained therein.

### Presentation of the invention

The present invention aims to overcome the aforementioned technical drawbacks by providing a method for the processing of Cannabis Sativa L. which makes it possible to control the yield of the finished product in each processing step.

In particular, the main object of the present invention is to provide a method for the processing of Cannabis Sativa L. suited to maintain high quality standards of the finished product.

A further object of the present invention is to provide a method for the processing of Cannabis Sativa L. which allows to maximize the amount of finished product that can be obtained from the harvest of the branches.

Another object of the present invention is to provide a method for the processing of Cannabis Sativa L. which is simple to implement.

A further object of the present invention is to provide a method for the processing of Cannabis Sativa L. with a limited impact on the equipment used in the industry to carry out the activities required in the flower treatment process.

These objectives, together with others that will be better clarified below, are achieved by a method for the processing of Cannabis Sativa L. of the type in accordance with claim 1.

Other objects that will be better described below are achieved by a method for the processing of Cannabis Sativa L. in accordance with the dependent claims.

### Detailed description of the invention

The present invention relates to a method for the processing of Cannabis Sativa L.

It should be noted that the cultivation of Cannabis Sativa L. and the processing of its flowers to obtain the molecule are practices recognized to be legal in a significant number of countries, including European countries and the United States.

The method which is the subject of the present invention comprises a step a) of destemming of the Cannabis Sativa L. flowers.

The separation of the flowers from the branch is carried out during the execution of said step a).

In a first embodiment, this step a) may be performed manually.

In this case, therefore, the destemming is carried out by an operator in one of the following ways:
i) the operator takes the flowers directly from the plant without carrying out a previous step of cutting the branches from the plant;
ii) the operator separates the flowers from the branches of the plant after carrying out a cutting step of branches from the latter (known as the harvesting step).

Therefore, when the destemming step a) is performed manually, this step may (or may not) be preceded by the execution of the harvesting step.

Alternatively, the destemming step a) may be carried out automatically or semiautomatically by means of special devices called, in the commercial jargon of the sector, *destemmers* or *bloomers.*

In particular, these devices are generally provided with holes or openings for the insertion of branches placed in communication with two or more motorized rollers (generally made of rubber) whose profile is shaped according to a determined geometry.

When rotating, said rollers mutually engage each other with play, so as to interact with the branches dragging them while simultaneously detaching the flowers.

At the outlet of said devices, it is possible to collect the flowers (intended to be subjected to the other steps of the processing method) and the branches, which on the contrary are a waste product, separately.

When the destemming step **a)** is carried out with the aid of specific devices or machines, it is always preceded by the harvesting step.

Conveniently, the method comprises a step b) of freezing the fresh Cannabis Sativa L. flowers.

This freezing step b) can be such as to facilitate the temporary storage of the Cannabis Sativa L. flowers in an environment at a temperature between -120°C to - 20°C.

In addition, during the execution of the freezing step b), the Cannabis Sativa L. flowers may be left in this environment for a period longer than 120 seconds.

In particular, when the temperature inside the environment intended for the freezing of the flowers is close to -120°C, the duration of step b) shall be about 120 seconds, while when the temperature inside the same environment is close to -20°C, the duration of step b) shall be about 24 hours (1 day).

Typically, the freezing step b) may have a duration of a few hours; moreover, the duration of this step may be extended depending on the needs to enable the storage of the frozen Cannabis Sativa L. flowers until the next step is carried out.

Conveniently, to freeze Cannabis Sativa L. flowers, cryogenic gases or fluids may be used which are introduced into the closed environment (which may be a tunnel of a predetermined length) where the Cannabis Sativa L. flowers are temporarily stored.

For example, the freezing step b) may be executed using a cryogenic fluid such as liquid nitrogen or similar.

In addition, the temporary storage environment of the Cannabis Sativa L. flowers may be constituted by a closed chamber or a tunnel of a predetermined length.

In the event that a tunnel is used, the Cannabis Sativa L. flowers may be moved mechanically (for example by means of a conveyor belt) so that they remain in the tunnel itself for a sufficient amount of time to allow them to be frozen.

Alternatively, the freezing of the Cannabis Sativa L. flowers during the execution of step b) may be obtained by subjecting the product to refrigeration cycles with CO₂ gas or by using standard refrigeration cells or even through the use of blast chillers (of the type similar to that already used in the food industry).

Conveniently, the method comprises a step c) of trimming the Cannabis Sativa L. flowers.

During the trimming step, the outer leaves of the Cannabis Sativa L. flower are removed in order to obtain the inner part of this flower (also known as the *heart*).

In the traditional and known methods for processing Cannabis Sativa L., the trimming is generally carried out with the aid of specific machines called *trimmers.*

In general, trimmers are equipped with blades suited to interact with the outer parts of the flower in order to remove the most peripheral leaves and to obtain the heart of the flower itself.

The purpose of the trimming step c) is to obtain a substantially compact and uniform flower of Cannabis Sativa L., that is, free of leaflets protruding from its core (or heart).

The method furthermore comprises a step d) of drying the Cannabis Sativa L. flower.

In particular, the drying step d) can be carried out in a specific and specially arranged environment at a predetermined pressure and temperature (hereinafter in the following description this pressure and this temperature can also be indicated with the expression *"ambient pressure"* and *"ambient temperature,"* where the adjective "ambient" is meant to refer to the environment in which the drying step d) is carried out).

Advantageously, the execution of the present method provides for a freezing step b) and a drying step d) which are suited to be distinct and separate from each other.

In particular, the freezing step b) and the drying step d) are not carried out simultaneously, as is generally required by the methods for processing Cannabis Sativa L. which involve cryodesiccation.

In other words, the pressure of the zone or chamber in which the freezing step b) is carried out is substantially constant and equal to the atmospheric pressure.

cryodesiccation, in fact, promotes the freeze-drying of Cannabis Sativa L. flower through the combined and simultaneous effect caused by freezing and applying a vacuum to the Cannabis Sativa L. flower.

During the cryodesiccation, the water molecules contained in the Cannabis Sativa L. flower undergo the direct transition from the solid phase (ice crystals) to the vapour phase (sublimation process).

During the execution of the cryodesiccation, the step involving the lowering of the temperature is also carried out along with the simultaneous lowering of the ambient pressure, since a reduced pressure favours the "cold" effect that is obtained on the Cannabis Sativa L. flower (in other words, the drop in pressure "amplifies" the freezing effect obtained on the flower).

The purpose of the cryodesiccation step is to remove the water molecules contained in the Cannabis Sativa L. flower, and toward that end it is common to carry out the cryodesiccation step together with the introduction of a reduced amount of heat inside the environment in which the Cannabis Sativa L. flowers are placed.

The method which is the subject of the present invention allows the same objectives of the cryodesiccation (that is, the controlled removal of the water molecules contained in the Cannabis Sativa L. flower) to be achieved through two separate steps, respectively the freezing step b) and the drying step d); however, these two steps are performed at different moments in time.

In other words, the drying step d) is carried out only after completion of the freezing step b).

For this reason, the execution of the freezing step b) and the drying step d) do not provide any overlapping, that is, with respect to their duration, it is never possible to define an instant or an interval of time wherein the two steps are simultaneously carried out.

Conversely, in a considerable number of cases between the end of a given step and the beginning of the next step many hours and/or days and/or weeks may also lapse (for example, when the freezing step b) is performed before the drying step d)).

Conveniently, the present method includes a single drying step d) which has a predetermined duration.

The single drying step d) is carried out in a single operation, that is, continuously and without interruptions throughout its duration.

Therefore, interruptions are not provided during the execution of the drying step d), which is thus performed in a single operation without the subdivision of the drying process into two or more sub-steps (each of which having a shorter duration than the entire drying step) carried out in different moments of time (that is, associating a pause between the end of one sub-step and the beginning of the following one).

In particular, the drying step d) is performed at a controlled temperature to promote the extraction of a significant part of the amount of water contained in the Cannabis Sativa L. flowers.

According to a particularly advantageous aspect of the present method, the drying step d) the Cannabis Sativa L. flowers is carried out in an environment designed for said step which has conditions subjected to a relatively high vacuum.

In particular, the environmental conditions adopted for the execution of the drying step d) are such as to promote a vacuum condition inside the environment suited to maintain a pressure lower than that corresponding to the triple point of water.

As is known, the triple point of water is found at a pressure of 0.6117 KPa, which corresponds to about 6 mbar absolute.

For this reason, the drying step d) can be carried out at an ambient pressure lower than 6 mbar absolute and generally not higher than 5 mbar absolute.

Conveniently, in a particular embodiment of the present method, the freezing step b) can always be performed before a drying step d) carried out under relatively high vacuum conditions.

The sequence of these two steps is important since by freezing the flowers it is possible to transform the water contained in them into ice crystals; in the subsequent drying step, the water contained inside the flowers in the form of ice crystals undergoes sublimation and is directly converted into water vapour.

The drying process under high vacuum, starting from frozen flowers, allows a high amount of water to be extracted from the latter through the direct passage to the sublimation phase (due to the operating conditions set below the triple point of water).

In addition, during the execution of the drying step d), a reduced amount of heat may be introduced (generally by irradiation) into the environment where the flowers are contained.

The amount of heat introduced into the environment where the flowers are collected and desiccated may be such as not to alter in a detectable manner the freezing condition to which the flowers are subject.

The partial heating of the environment favours the direct sublimation of the ice crystals into vapour with the benefit of maximizing the amount of water extracted from the Cannabis Sativa L. flowers in a short time.

Conveniently, the drying step d) under relatively high vacuum conditions may have a predetermined duration, generally between 5 and 48 hours.

Preferably, the drying step d) under relatively high vacuum conditions may have a duration between 5 and 10 hours.

From the above, during the execution of the drying step d) in the environment suitably arranged for this step there are a predetermined ambient pressure and ambient temperature.

Preferably, the execution of the drying step d) may take place in such a way as to:
- promote the variation of the ambient pressure according to a predetermined trend;
- promote the variation of the ambient temperature according to a predetermined trend.

In other words, in the time interval that defines the duration of the drying step d), the ambient pressure and the ambient temperature may vary according to a predetermined trend.

The trend of the curve followed by the ambient temperature and pressure may be different (for example, increasing, decreasing, partly increasing and partly decreasing) and may be substantially linear or follow curves of known functions (for example, parabolic, hyperbolic, Gaussian, etc.).

Typically, during the execution of the drying step d), the trend of the curves associated with the ambient pressure and the ambient temperature may be substantially decreasing, and in particular of the gradually decreasing type.

In this case, at the beginning of the execution of the drying step d), the value of the ambient pressure and the ambient temperature shall be substantially maximum and equal to a predetermined value.

At the end of the drying step d) (i.e., after the entire duration of the same), the value of the ambient pressure and the ambient temperature shall be substantially minimum and equal to a predetermined value.

From an operational standpoint, the ambient pressure inside the environment in which the drying step d) is carried out shall be adjusted through special pressure regulating means, just as the temperature inside the same environment shall be adjusted by changing the amount of heat introduced into said environment overtime.

In general, during the drying step d):
- the maximum predetermined value of the ambient pressure may be between 2 mbar absolute and 3 mbar absolute;
- the minimum predetermined value of the ambient pressure may be between 0.1 mbar absolute and 0.5 mbar absolute;
- the maximum predetermined value of the ambient temperature may be between 50°C absolute and 80°C;
- the minimum predetermined value of the ambient temperature may be between 15°C and 20°C.

The following table intends to provide an example of how the ambient pressure and the ambient temperature may vary during the execution of the drying step d):

| *Unit of measurement* | *drying step d)* | | | | | | | | | |
|---|---|---|---|---|---|---|---|---|---|---|
| Hours | 1 | 2 | 3 | 4 | 5 | 6 | 7 | 8 | 9 | 10 |
| mbar absolute | 2 | 2 | 2 | 2 | 1 | 0.5 | 0.5 | 0.2 | 0.2 | 0.2 |
| °C | 70 | 70 | 70 | 70 | 50 | 50 | 30 | 20 | 20 | 20 |
| °C | -20 | -15 | -10 | -5 | 0 | 0 | 5 | 10 | 15 | 20 |

In the example shown in the table, the drying step has a total duration of 10 hours (second line).

The third line indicates the variation of the ambient pressure during the execution of the drying step d).

The ambient pressure values are determined with an hourly interval so as to obtain ten discrete values of the ambient pressure during the execution of the drying step.

The fourth line indicates the variation of the ambient temperature during the execution of the drying step d).

Also, the ambient temperature values are determined with an hourly interval so as to obtain ten discrete temperature values during the execution of the drying step.

As can be clearly seen from the table above, the ambient pressure and the ambient temperature decrease progressively from their respective initial maximum values (2 mbar absolute/70°C) and their respective final minimum values (0.2 mbar/20°C).

In addition, it is possible to approximate the pressure and temperature curves with a substantially linear trend.

The fifth line indicates the temperature trend of the Cannabis Sativa L. flowers subjected to the drying step d) with the temperatures/pressures indicated in the second and third line of the table.

The temperature trend associated with the Cannabis Sativa L. flowers also varies between a maximum and a minimum (respectively -20°C and 20°C) with a substantially linear trend that essentially follows that of the ambient temperature.

In addition, the Cannabis Sativa L. flowers subjected to the drying step d) described in the table were also previously subjected to a freezing step b).

Preferably, following the execution of the drying step d) described in the present method, the Cannabis Sativa L. flowers may have a residual water content in the range of 8% - 20% with respect to the total weight of the flowers themselves.

Thus, the duration of the drying step d), as well as the minimum and maximum ambient pressure and temperature, can be selected to obtain, at the end of this step, a water content on the Cannabis Sativa L. flower ranging between 8% and 20%.

In this regard, it was possible to observe experimentally how, in a drying step with a maximum duration of 10 hours, the minimum and maximum ambient pressure/temperature must be selected within the range previously indicated in paragraph [0089].

These values of duration, pressure and temperature are also valid in the case where the Cannabis Sativa L. flowers have an initial temperature (before the drying step d)) between -30°C and -20°C and a final temperature (at the end of the drying step d)) between 15°C and 30°C.

In addition, during the execution of the drying step d), it is possible to carry out a removal step e) of the water vapour present in the environment and extracted from the Cannabis Sativa L. flowers and a subsequent step f) of condensation of said vapour.

Step d) may involve the use of vacuum pumps designed to remove water vapour from the flowers; these pumps may be connected to a Freon-based circulation condenser (or based on ammonia or cryogenic gases) generally installed outside the drying chamber and suited to carry out step **f)** in order to recondense the water previously extracted from the flowers.

The execution of step d) is substantially aimed at promoting the freeze-drying of the Cannabis Sativa L. flowers.

In substance, step d) allows the application of the freeze-drying technique (already commonly used in the pharmaceutical and food industries) also for the processing of Cannabis Sativa L. flowers.

The advantage of applying a drying step d) in environmental conditions of high vacuum lies in the possibility of promoting a significant extraction of the water contained in the flowers; in fact, the vacuum condition allows to increase the degree of final drying to which the flowers of Cannabis Sativa L. processed with the present method.

It is therefore clear that steps a) to d) of the method described above allow to improve the processing of Cannabis Sativa L. flowers, to maintain their original colour, to promote long-term preservation, and to reduce the risk that the water present in them may accelerate their deterioration.

The present method also enables to complete the entire processing of the Cannabis Sativa L. flowers in a relatively short time (typically less than 10 hours) while obtaining a high quality product in terms of i) control of the amount of residual water contained therein, *ii)* degree of colouring similar to the original colour before the application of the method, and *iii)* maintenance of the original scent.

The steps of the method described above can be carried out in a strict and sequential order, better described in the examples below:

### Example 1

| | |
|---|---|
| First step: | **a)** destemming of the Cannabis Sativa L. flowers; |
| Second step: | **b)** freezing of the Cannabis Sativa L. flowers; |
| Third step: | **c)** trimming of the Cannabis Sativa L. flowers; |
| Fourth step: | **d)** drying of the Cannabis Sativa L. flowers. |

### Example 2

| | |
|---|---|
| First step: | **a)** destemming of the Cannabis Sativa L. flowers; |
| Second step: | **c)** trimming of the Cannabis Sativa L. flowers; |
| Third step: | **b)** freezing of the Cannabis Sativa L. flowers; |
| Fourth step: | **d)** drying of the Cannabis Sativa L. flowers. |

### Example 3

| | |
|---|---|
| First step: | **a)** destemming of the Cannabis Sativa L. flowers; |
| Second step: | **b)** freezing of the Cannabis Sativa L. flowers; |
| Third step: | **d)** drying of the Cannabis Sativa L. flowers; |
| Fourth step: | **c)** trimming of the Cannabis Sativa L. flowers. |

In the case of *Example* 2 and *Example 3,* the freezing step b) and the drying step d) are carried out not only in this order (as described above) but also sequentially, that is, without interposing any other step of the method therebetween.

In *Example 2,* the trimming step c) is carried out on fresh flowers, that is, before they are dried.

Therefore, in this case the trimming step is performed in the "green" condition, that is, with the Cannabis Sativa L. flower substantially in the same condition as when it was harvested. Before this step, in fact, only the destemming process is carried out in order to separate the Cannabis Sativa L. flowers from the branches of the plant.

The drying step **d)** is then carried out on the already destemmed flowers, and therefore this facilitates the substantial freeze-drying of the heart of these flowers.

Alternatively, following the order of the steps indicated in *Example* 1 and *Example 3,* it is possible to find a trimming step **c)** carried out after the freezing step **b)** (and in the case illustrated in *Example 3,* the trimming of the flowers is carried out after performing both the freezing and the drying step).

In this case, the trimming process is carried out on the dry flowers, that is, already free of water (or with a very low moisture content).

The execution of a trimming step c) of the Cannabis Sativa L. flowers carried out in "green" or "dried" conditions requires the use of trimmers having different types of blades or tools; it is in fact clear that the mechanical characteristics of the flower vary significantly depending on whether it is fresh or dried.

In fact, it has been found experimentally, after numerous tests, that the freezing step **b)** makes the outer leaves of the flower particularly fragile, and that these leaves can be easily removed later in the trimming step c) by using conveniently adapted trimmers.

The preventive freezing of the flowers also keeps the outer leaves of the latter in a "semi-crystalline" state or with a consistency that is slightly higher with respect to when the flowers are dried and much lower with respect to when they are still fresh.

In this particular condition, the execution of the trimming step c) can be carried out in a particularly precise manner, enabling the complete and selective removal of the outer leaves of the flower while at the same time keeping the heart of the latter perfectly preserved.

Finally, the method may include the execution of a step **g)** of storage of the Cannabis Sativa L. flowers.

The storage step **g)** is carried out at the end of all the aforementioned steps **a)** to **d)** and may involve the storage of the product in boxes and/or containers pending its sale or before it is subjected to further processing steps suited to promote the extraction of the CBD and/or terpenes.

The present invention can be implemented in other variants, all of which fall within the scope of the inventive characteristics claimed and described herein; these technical characteristics may be replaced by different but technically equivalent elements and materials; the forms and dimensions of the invention can be any, as long as they are compatible with its use.

The letters and any reference signs included in the claims and in the description have the sole purpose of facilitating the comprehension of the explanations and must not be considered elements that limit the technical interpretation of the objects or processes indicated by them.

## Claims

1. A method for the processing of Cannabis Sativa L., comprising the following steps:
a) destemming of the flowers of Cannabis Sativa L.;
b) freezing of the flowers of Cannabis Sativa L.;
c) trimming of the flowers of Cannabis Sativa L.;
d) drying of the flowers of Cannabis Sativa L.;
wherein said destemming step a) is suited to separate the flowers of Cannabis Sativa L. from the branches;
wherein said freezing step b) is performed in an environment with a temperature included between -120°C and -20°C and lasts more than 120 seconds;
wherein said trimming step c) is suited to promote the removal of the outermost leaves of each flower of Cannabis Sativa L.;
wherein said step d) of drying the flowers of Cannabis Sativa L. is carried out under high vacuum environmental conditions at a pressure of less than 6 mbar absolute to promote the sublimation into vapour of the water contained in the flowers of Cannabis Sativa L.
and wherein said freezing step b) and said drying step d) are different and separate; said drying step d) being carried out after said freezing step b) once the latter has been completed and finished.

2. Method according to claim 1, **characterized in that** it comprises a single drying step d) having a predetermined duration, said single drying step being continuous and without interruptions between the initial instant and the final instant of said duration.

3. Method according to claim 1 or 2, **characterized in that** said drying step d) under vacuum conditions has a duration included between 5 hours and 48 hours, and preferably included between 5 and 10 hours.

4. Method according to claim 3, **characterized in that**, for the duration of said drying step d), the pressure in the environment in which said step is carried out varies according to a predetermined trend.

5. Method according to claim 3 or 4, **characterized in that**, for the duration of said drying step d), the temperature in the environment where said step is carried out varies according to a predetermined trend.

6. Method according to claim 4 or 5, **characterized in that**, during the execution of said drying step d), the pressure has a gradually decreasing trend between a predetermined maximum and a predetermined minimum value.

7. Method according to claim 4 or 5, **characterized in that**, during the execution of said drying step d), the temperature has a gradually decreasing trend between a predetermined maximum and a predetermined minimum value.

8. Method according to claim 6, **characterized in that** said predetermined minimum value of said pressure is included between 0.3 mbar and 0.1 mbar and said predetermined maximum value of said pressure is included between 3 mbar and 2 mbar.

9. Method according to claim 7, **characterized in that** said predetermined minimum value of said temperature is included between 15°C and 25°C and said predetermined maximum value of said temperature is included between 60°C and 70°C.

10. Method according to one or more of the preceding claims, **characterized in that** the duration of said drying step d) and the selection of said, respectively, predetermined maximum and minimum values of pressure and temperature are selected in such a way as to bring the flowers of Cannabis Sativa L. to a temperature included between -30°C and 30°C with a water content included between 8% and 20% of the total weight of the flowers.

11. Method according to one or more of the preceding claims, **characterized in that** said destemming step a) is carried out before said freezing step b), said freezing step b) being carried out before said trimming step c), said trimming step c) being carried out before said drying step d) under vacuum conditions.

12. Method according to one or more of the preceding claims, **characterized in that** said destemming step a) is carried out before said trimming step c), said trimming step c) being carried out before said freezing step b), said freezing step b) being carried out before said drying step d) under vacuum conditions.

13. Method according to one or more of the preceding claims, **characterized in that** said destemming step a) is carried out before said freezing step b), said freezing step b) being carried out before said drying step d) under vacuum conditions, said drying step d) under vacuum conditions being carried out before said trimming step c).

14. Method according to one or more of the preceding claims, **characterized in that** it comprises a step e) of removal of the water vapour present in the environment and extracted from the flowers of Cannabis Sativa L. during the execution of said drying step d), said method also comprising a step f) of condensation of the water vapour removed from the flowers of Cannabis Sativa during the execution of said drying step d).

15. Method according to one or more of the preceding claims, **characterized in that** it comprises a step g) of storing of the flowers of Cannabis Sativa L..
